(19) Europäisches Patentamt
European Patent Office
Office européen des brevets

(11) **EP 3 760 207 A1**

(12) **EUROPEAN PATENT APPLICATION**
published in accordance with Art. 153(4) EPC

(43) Date of publication:
**06.01.2021 Bulletin 2021/01**

(21) Application number: **19759990.5**

(22) Date of filing: **27.02.2019**

(51) Int Cl.:
***A61K 31/7072*** *(2006.01)* ***A61K 9/08*** *(2006.01)*
***A61K 9/10*** *(2006.01)* ***A61K 47/32*** *(2006.01)*
***A61K 47/34*** *(2017.01)* ***A61K 47/36*** *(2006.01)*
***A61K 47/42*** *(2017.01)* ***A61P 27/02*** *(2006.01)*

(86) International application number:
**PCT/JP2019/007542**

(87) International publication number:
**WO 2019/168023 (06.09.2019 Gazette 2019/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.02.2018 JP 2018035578**

(71) Applicant: **Santen Pharmaceutical Co., Ltd.**
**Kita-ku**
**Osaka-shi**
**Osaka 530-8552 (JP)**

(72) Inventors:
- **TAKAHASHI, Kyohei**
  **Ikoma-shi, Nara 630-0101 (JP)**
- **ASADA, Hiroyuki**
  **Ikoma-shi, Nara 630-0101 (JP)**
- **KAMIMURA, Asuka**
  **Ikoma-shi, Nara 630-0101 (JP)**
- **MORISHIMA, Kenji**
  **Ikoma-shi, Nara 630-0101 (JP)**
- **MOMOKAWA, Yusuke**
  **Ikoma-shi, Nara 630-0101 (JP)**
- **ENDO, Kenichi**
  **Ikoma-shi, Nara 630-0101 (JP)**

(74) Representative: **Müller-Boré & Partner Patentanwälte PartG mbB**
**Friedenheimer Brücke 21**
**80639 München (DE)**

# (54) OPHTHALMIC COMPOSITION COMPRISING DIQUAFOSOL AND CATIONIC POLYMER

(57) Provided is an ophthalmic composition comprising diquafosol or a salt thereof and a cationic polymer, the cationic polymer being at least one selected from the group consisting of chitosan, a chitosan derivative, a cationic (meth)acrylate copolymer, a cationic silicone polymer, a diallyl quaternary ammonium salt-acrylamide copolymer, cationic hydrolyzed keratin, cationic hydrolyzed silk, cationic hydrolyzed collagen, cationic hydrolyzed casein, cationic hydrolyzed soy protein, a cationic vinylpyrrolidone copolymer, polyvinylpyrrolidone, a dimethyldiacrylammonium chloride homopolymer, an adipic acid-dimethylaminohydroxypropyldiethylenetriamine copolymer, an adipic acid-epoxypropyldiethylenetriamine copolymer and an acrylamide-β-methacryloyloxyethyltrimethylammoniummethyl sulfate copolymer.

FIG.1

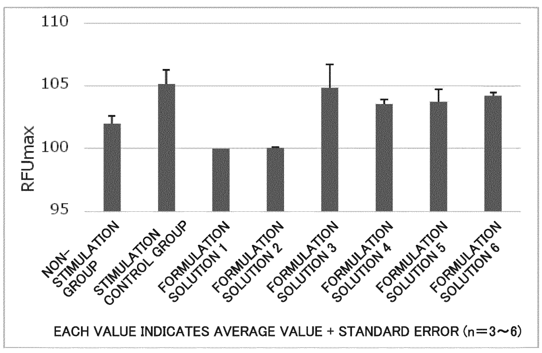


EP 3 760 207 A1

## Description

TECHNICAL FIELD

The present invention relates to an ophthalmic composition comprising diquafosol or a salt thereof and a cationic polymer.

BACKGROUND ART

The diquafosol is a purinoceptor agonist called $P^1$,$P^4$-di(uridine-5') tetraphosphate or Up4U, has a tear secretion promoting action, and is used for treatment of dry eye as an ophthalmic solution containing a diquafosol tetrasodium salt at a concentration of 3% (w/v) (product name: Diquas (registered trademark) Ophthalmic Solution 3%) (PTL 1 and NPL 1). The diquafosol tetrasodium salt is extremely easily soluble in water, and Diquas (registered trademark) Ophthalmic Solution 3% is a colorless and clear sterile aqueous eye drop (NPL 1).

On the other hand, the cationic polymer refers to a polymer containing one or more substituents which turn into cations when the polymer is dissolved in water. The cationic polymer is used in various applications. For example, Japanese Patent Laying-Open No. 2006-321757 (PTL 2) describes that the cationic polymer exhibits a preventive effect against a squeaking feeling, a sticky feeling and entanglement at the time when hair is wet, i.e. in a stage which begins with washing off a hairdressing detergent, passes with treatment with shampoo, etc., and ends with drying the hair, and the cationic polymer also contributes to the effect of imparting gloss, softness, smoothness, a moist feeling, ease of arrangement and the like to hair in drying.

Polyvinylpyrrolidone, which is a type of cationic polymer, is used as, for example, a suspending agent or a solubilizing agent for hardly soluble compounds in the field of ophthalmic compositions (PTL 3).

CITATION LIST

PATENT LITERATURE

PTL 1: Japanese Patent No. 3652707
PTL 2: Japanese Patent Laying-Open No. 2006-321757
PTL 3: Japanese Patent Laying-Open No. H01-294620

NON PATENT LITERATURE

NPL 1: Package Insert of Diquas (registered trademark) Ophthalmic Solution 3%

SUMMARY OF INVENTION

TECHNICAL PROBLEM

An object of the present invention is to discover a novel ophthalmic composition comprising diquafosol or a salt thereof.

SOLUTION TO PROBLEM

The present inventors have extensively conducted studies, and resultantly discovered an ophthalmic composition comprising diquafosol or a salt thereof and a cationic polymer, the cationic polymer being at least one selected from the group consisting of chitosan, a chitosan derivative, a cationic (meth)acrylate copolymer, a cationic silicone polymer, a diallyl quaternary ammonium salt-acrylamide copolymer, cationic hydrolyzed keratin, cationic hydrolyzed silk, cationic hydrolyzed collagen, cationic hydrolyzed casein, cationic hydrolyzed soy protein, a cationic vinylpyrrolidone copolymer, polyvinylpyrrolidone, a dimethyldiacrylammonium chloride homopolymer, an adipic acid-dimethylaminohydroxypropyldiethylenetriamine copolymer, an adipic acid-epoxypropyldiethylenetriamine copolymer and an acrylamide-β-methacryloyloxyethyltrimethylammoniummethyl sulfate copolymer, with the findings that the ophthalmic composition has a high tear volume increasing action and that the cationic polymer has a metabolic stability effect on diquafosol and a salt thereof. Further, the present inventors have found that an ophthalmic composition comprising diquafosol or a salt thereof and polyvinylpyrrolidone which is a type of cationic polymer does not exhibit neurostimulatory, enables improvement of the pouring touch of the ophthalmic solution, and has a high tear volume increasing action. Hereinafter, these compositions are each also referred to as "the present composition". That is, the present invention relates to the following.

The present composition is an ophthalmic composition comprising diquafosol or a salt thereof and a cationic polymer, the cationic polymer being at least one selected from the group consisting of chitosan, a chitosan derivative, a cationic (meth)acrylate copolymer, a cationic silicone polymer, a diallyl quaternary ammonium salt-acrylamide copolymer, cationic hydrolyzed keratin, cationic hydrolyzed silk, cationic hydrolyzed collagen, cationic hydrolyzed casein, cationic hydrolyzed soy protein, a cationic vinylpyrrolidone copolymer, polyvinylpyrrolidone, a dimethyldiacrylammonium chloride homopolymer, an adipic acid-dimethylaminohydroxypropyldiethylenetriamine copolymer, an adipic acid-epoxypropyldiethylenetriamine copolymer and an acrylamide-p-methacryloyloxyethyltrimethylammoniummethyl sulfate copolymer.

In the present composition, the cationic polymer is preferably at least one selected from the group consisting of chitosan, a chitosan derivative, a diallyl quaternary ammonium salt-acrylamide copolymer and polyvinylpyrrolidone.

In the present composition, the cationic polymer is preferably polyvinylpyrrolidone.

Preferably, the present composition comprises polyvinylpyrrolidone having a K value of 17 or more.

Preferably, the present composition comprises polyvinylpyrrolidone having a K value of 17 to 90.

Preferably, the present composition comprises polyvinylpyrrolidone having a K value of 30.

Preferably, the present composition comprises polyvinylpyrrolidone having a K value of 90.

In the present composition, the cationic polymer is preferably at least one selected from the group consisting of chitosan, a chitosan derivative.

In the present composition, the concentration of the cationic polymer is preferably 0.00001 to 10% (w/v).

In the present composition, the concentration of the diquafosol or a salt thereof is preferably 0.0001 to 10% (w/v).

In the present composition, the concentration of the diquafosol or a salt thereof is more preferably 0.01 to 5% (w/v).

In the present composition, the concentration of the diquafosol or a salt thereof is still more preferably 1 to 5% (w/v).

In the present composition, the concentration of the diquafosol or a salt thereof is furthermore preferably 3% (w/v).

The present composition is preferably an eye drop.

The present composition is preferably aqueous.

The present composition is preferably a suspension-type or solution-type composition.

The viscosity of the present composition is preferably 1 to 500 mPa·s at 25°C.

The viscosity of the present composition is more preferably 1 to 100 mPa·s at 25°C.

In the present composition, the salt of the diquafosol is preferably diquafosol sodium.

The present composition is preferably a composition for prevention or treatment of dry eye.

Preferably, the present composition is instilled into an eye 1 to 6 times a day in a dose of 1 to 5 drops each time.

More preferably, the present composition is instilled into an eye 2 to 4 times a day in a dose of 1 or 2 drops each time.

Still more preferably, the present composition is instilled into an eye 3 or 4 times a day in a dose of 1 or 2 drops each time.

The present invention also provides an ophthalmic composition comprising diquafosol or a salt thereof and polyvinylpyrrolidone.

Preferably, the present composition comprises polyvinylpyrrolidone having a K value of 17 or more.

Preferably, the present composition comprises polyvinylpyrrolidone having a K value of 17 to 90.

Preferably, the present composition comprises polyvinylpyrrolidone having a K value of 30.

Preferably, the present composition comprises polyvinylpyrrolidone having a K value of 90.

The present invention also provides a therapeutic agent for dry eye, comprising diquafosol or a salt thereof and a cationic polymer. Here, the cationic polymer is at least one selected from the group consisting of chitosan, a chitosan derivative, a cationic (meth)acrylate copolymer, a cationic silicone polymer, a diallyl quaternary ammonium salt-acrylamide copolymer, cationic hydrolyzed keratin, cationic hydrolyzed silk, cationic hydrolyzed collagen, cationic hydrolyzed casein, cationic hydrolyzed soy protein, a cationic vinylpyrrolidone copolymer, polyvinylpyrrolidone, a dimethyldiacrylammonium chloride homopolymer, an adipic acid-dimethylaminohydroxypropyldiethylenetriamine copolymer, an adipic acid-epoxypropyldiethylenetriamine copolymer and an acrylamide-β-methacryloyloxyethyltrimethylammoniummethyl sulfate copolymer.

The present invention also provides a therapeutic agent for dry eye, comprising diquafosol or a salt thereof and polyvinylpyrrolidone.

The present invention also provides a method for preventing or treating dry eye, comprising administering to a patient an ophthalmic composition comprising diquafosol or a salt thereof and a cationic polymer. Here, the cationic polymer is at least one selected from the group consisting of chitosan, a chitosan derivative, a cationic (meth)acrylate

copolymer, a cationic silicone polymer, a diallyl quaternary ammonium salt-acrylamide copolymer, cationic hydrolyzed keratin, cationic hydrolyzed silk, cationic hydrolyzed collagen, cationic hydrolyzed casein, cationic hydrolyzed soy protein, a cationic vinylpyrrolidone copolymer, polyvinylpyrrolidone, a dimethyldiacrylammonium chloride homopolymer, an adipic acid-dimethylaminohydroxypropyldiethylenetriamine copolymer, an adipic acid-epoxypropyldiethylenetriamine copolymer and an acrylamide-β-methacryloyloxyethyltrimethylammoniummethyl sulfate copolymer.

The present invention also provides a method for preventing or treating dry eye, comprising administering to a patient an ophthalmic composition comprising diquafosol or a salt thereof and polyvinylpyrrolidone.

The present invention also provides an ophthalmic composition for use in prevention or treatment of dry eye, the ophthalmic composition comprising diquafosol or a salt thereof and a cationic polymer. Here, the cationic polymer is at least one selected from the group consisting of chitosan, a chitosan derivative, a cationic (meth)acrylate copolymer, a cationic silicone polymer, a diallyl quaternary ammonium salt-acrylamide copolymer, cationic hydrolyzed keratin, cationic hydrolyzed silk, cationic hydrolyzed collagen, cationic hydrolyzed casein, cationic hydrolyzed soy protein, a cationic vinylpyrrolidone copolymer, polyvinylpyrrolidone, a dimethyldiacrylammonium chloride homopolymer, an adipic acid-dimethylaminohydroxypropyldiethylenetriamine copolymer, an adipic acid-epoxypropyldiethylenetriamine copolymer and an acrylamide-β-methacryloyloxyethyltrimethylammoniummethyl sulfate copolymer.

The present invention also provides an ophthalmic composition for use in prevention or treatment of dry eye, the ophthalmic composition comprising diquafosol or a salt thereof and polyvinylpyrrolidone.

The present invention also provides use of an ophthalmic composition for the manufacture of a medicament for preventing or treating dry eye, the ophthalmic composition comprising diquafosol or a salt thereof and a cationic polymer. Here, the cationic polymer is at least one selected from the group consisting of chitosan, a chitosan derivative, a cationic (meth)acrylate copolymer, a cationic silicone polymer, a diallyl quaternary ammonium salt-acrylamide copolymer, cationic hydrolyzed keratin, cationic hydrolyzed silk, cationic hydrolyzed collagen, cationic hydrolyzed casein, cationic hydrolyzed soy protein, a cationic vinylpyrrolidone copolymer, polyvinylpyrrolidone, a dimethyldiacrylammonium chloride homopolymer, an adipic acid-dimethylaminohydroxypropyldiethylenetriamine copolymer, an adipic acid-epoxypropyldiethylenetriamine copolymer and an acrylamide-β-methacryloyloxyethyltrimethylammoniummethyl sulfate copolymer.

The present invention also provides use of an ophthalmic composition for the manufacture of a medicament for preventing or treating dry eye, the ophthalmic composition comprising diquafosol or a salt thereof and polyvinylpyrrolidone.

## ADVANTAGEOUS EFFECTS OF INVENTION

As is evident from the test results described later, the present composition has a high tear volume increasing action. Further, the cationic polymer contained in the present composition has a metabolic stability effect on diquafosol or a salt thereof. Hence, the present composition is expected to exhibit a more potent therapeutic effect on dry eye as compared to a case where an existing Diquas (registered trademark) Ophthalmic Solution is instilled into an eye. The existing Diquas (registered trademark) Ophthalmic Solution is required to be instilled into an eye 6 times a day, and some patients are unable to obtain an expected effect due to poor adherence to instillation. The present composition is expected to improve adherence to instillation with the instillation frequency reduced while exhibiting a sufficient therapeutic effect on dry eye. Further, the existing Diquas (registered trademark) Ophthalmic Solution contains a diquafosol tetrasodium salt at a concentration of 3% (w/v), whereas the present composition is expected to exhibit an equivalent or more potent therapeutic effect on dry eye with a lower concentration. An ophthalmic composition comprising diquafosol or a salt thereof and polyvinylpyrrolidone which is a type of cationic polymer does not exhibit neurostimulatory, and therefore enables improvement of the pouring touch of the ophthalmic solution.

## BRIEF DESCRIPTION OF DRAWINGS

Fig. 1 is a graph showing a maximum fluorescence intensity (RFUmax) after addition of diquafosol sodium.

## DESCRIPTION OF EMBODIMENTS

The present invention will be described in more detail.

The term "(w/v) %" as used herein means a mass (g) of an intended ingredient contained in 100 mL of an ophthalmic composition of the present invention.

The "diquafosol" is a compound of the following chemical structural formula.

[Formula 1]

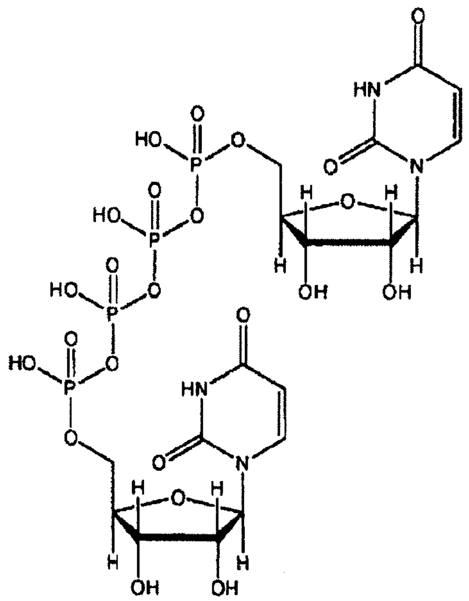

The "salt of diquafosol" is not particularly limited as long as it is a pharmaceutically acceptable salt, and examples thereof include salts with metals such as lithium, sodium, potassium, calcium, magnesium and zinc; salts with inorganic acids such as hydrochloric acid, hydrobromic acid, hydroiodic acid, nitric acid, sulfuric acid and phosphoric acid; salts with organic acids such as acetic acid, fumaric acid, maleic acid, succinic acid, citric acid, tartaric acid, adipic acid, gluconic acid, glucoheptonic acid, glucuronic acid, terephthalic acid, methanesulfonic acid, lactic acid, hippuric acid, 1,2-ethanesisulfonic acid, isethionic acid, lactobionic acid, oleic acid, pamoic acid, polygalacturonic acid, stearic acid, tannic acid, trifluoromethanesulfonic acid, benzenesulfonic acid, p-toluenesulfonic acid, lauryl sulfate, methyl sulfate, naphthalenesulfonic acid and sulfosalicylic acid; quaternary ammonium salts with methyl bromide, methyl iodide and the like; salts with halogen ions such as bromine ions, chlorine ions and iodine ions; salts with ammonia; and salts with organic amines such as triethylenediamine, 2-aminoethanol, 2,2-iminobis(ethanol), 1-deoxy-1-(methylamino)-2-D-sorbitol, 2-amino-2-(hydroxymethyl)-1,3-propanediol, procaine and N,N-bis(phenylmethyl)-1,2-ethanediamine.

In the present invention, the "diquafosol or salts thereof" include hydrates and organic solvates of diquafosol (free body) or salts thereof.

When the "diquafosol or salts thereof" have crystal polymorphs and crystal polymorph groups (crystal polymorph systems), the crystal polymorphs and crystal polymorph groups (crystal polymorph systems) are also within the scope of the present invention. Here, the crystal polymorph group (crystal polymorph system) means individual crystal forms in various stages where crystal forms are changed according to the conditions and states of production, crystallization and storage of the crystals, and the entire process of the stages.

As the "diquafosol or a salt thereof" according to the present invention, a sodium salt of diquafosol is preferable, and a diquafosol tetrasodium salt of the following chemical structural formula (herein, also referred to simply as "diquafosol sodium") is especially preferable.

[Formula 2]

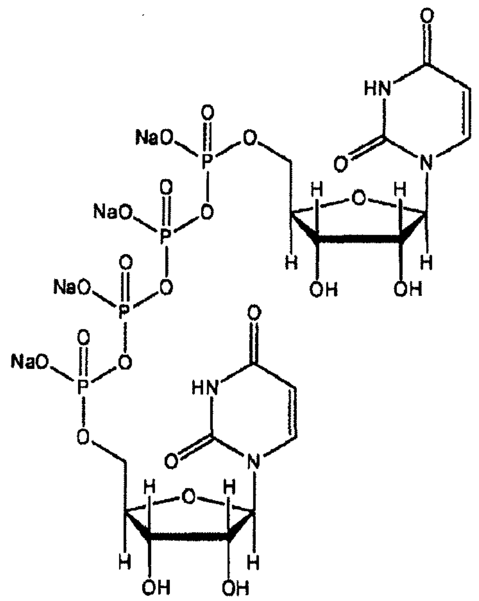

The diquafosol or salt thereof can be produced through, for example, the method disclosed in Japanese National Patent Publication No. 2001-510484.

The present composition may further contain active ingredients other than diquafosol or a salt thereof, or may contain diquafosol or a salt thereof as a single active ingredient.

The concentration of the diquafosol or salt thereof in the present composition is not particularly limited, and is, for example, preferably 0.0001 to 10% (w/v), more preferably 0.001 to 5% (w/v), still more preferably 0.01 to 5% (w/v), furthermore preferably 0.1 to 5% (w/v), furthermore preferably 1 to 5% (w/v), especially preferably 3% (w/v). More specifically, the concentration of the diquafosol or salt thereof in the present composition is 0.001% (w/v), 0.002% (w/v), 0.003% (w/v), 0.004% (w/v), 0.005% (w/v), 0.006% (w/v), 0.007% (w/v), 0.008% (w/v), 0.009% (w/v), 0.01% (w/v), 0.02% (w/v), 0.03% (w/v), 0.04% (w/v), 0.05% (w/v), 0.06% (w/v), 0.07% (w/v), 0.08% (w/v), 0.09% (w/v), 0.1% (w/v), 0.2% (w/v), 0.3% (w/v), 0.4% (w/v), 0.5% (w/v), 0.6% (w/v), 0.7% (w/v), 0.8% (w/v), 0.9% (w/v), 1% (w/v), 1.5% (w/v), 2% (w/v), 2.5% (w/v), 3% (w/v), 3.5% (w/v), 4% (w/v), 4.5% (w/v) or 5% (w/v).

In the present invention, "the cationic polymer" refers to a polymer containing one or more substituents which turn into cations when the polymer is dissolved in water.

The substituents in the cationic polymer, which turn into cations when the polymer is dissolved in water, are not particularly limited, and examples thereof include primary, secondary or tertiary amino groups, imino groups, imide groups, amide groups and quaternary ammonium groups.

The cationic polymer is not particularly limited, and is, for example, at least one selected from the group consisting of chitosan, a chitosan derivative, a cationic (meth)acrylate copolymer, a cationic silicone polymer, a diallyl quaternary ammonium salt-acrylamide copolymer, cationic hydrolyzed keratin, cationic hydrolyzed silk, cationic hydrolyzed collagen, cationic hydrolyzed casein, cationic hydrolyzed soy protein, a cationic vinylpyrrolidone copolymer, polyvinylpyrrolidone, a dimethyldiacrylammonium chloride homopolymer, an adipic acid-dimethylaminohydroxypropyldiethylenetriamine copolymer, an adipic acid-epoxypropyldiethylenetriamine copolymer and an acrylamide-β-methacryloyloxyethyltrimethylammoniummethyl sulfate copolymer. In particular, the cationic polymer in the present composition is preferably at least one selected from the group consisting of chitosan, a chitosan derivative, a diallyl quaternary ammonium salt-acrylamide copolymer and polyvinylpyrrolidone, more preferably at least one selected from the group consisting of chitosan, a chitosan derivative and polyvinylpyrrolidone.

The chitosan is a polysaccharide substantially composed of (A) monomeric β(1→4)-D-glucosamine bond units and (B) monomeric β(1→4)-N-acetyl-glucosamine bond units. Here, the numerical ratio of units (A) and units (B) is preferable such that the ratio of units (A) is about 50% to about 99% and the ratio of units (B) is about 1% to about 50%. Here, the numerical ratio of units (A) is also referred to as a "degree of deacetylation". The viscosity estimate of a 1% aqueous solution of chitosan is preferably about 1 mPa·s to about 3,000 mPa·s.

The chitosan includes chitosan salts such as hydrochlorides of chitosan.

Examples of the chitosan derivative include chitosan-N-acetylcysteine.

Examples of the cationic (meth)acrylate copolymer include vinylpyrrolidone-alkyldialkylamino (meth)acrylate

copolymers, quaternized derivatives from a vinylpyrrolidone-dimethylamino methacrylate copolymer and dimethyl sulfate, and aminoethyl acrylate phosphate-(meth)acrylate copolymers.

Examples of the cationic silicone polymer include cationic siloxane derivatives.

Examples of the diallyl quaternary ammonium salt-acrylamide copolymer include dimethyldiallylammonium chloride-acrylamide copolymers.

Examples of the cationic hydrolyzed keratin include N-[2-hydroxy-3-(trimethylammonio)propyl] chloride hydrolyzed keratin.

Examples of the cationic hydrolyzed silk include N-[2-hydroxy-3-(cocoalkyldimethylammonio)propyl] chloride hydrolyzed silk.

Examples of the cationic hydrolyzed collagen include N-[2-hydroxy-3-(cocoalkyldimethylammonio)propyl] chloride hydrolyzed collagen.

Examples of the cationic hydrolyzed casein include N-[2-hydroxy-3-(cocoalkyldimethylammonio)propyl] chloride hydrolyzed casein.

Examples of the cationic hydrolyzed soy protein include N-[2-hydroxy-3-(cocoalkyldimethylammonio)propyl] chloride hydrolyzed soy protein.

Examples of the cationic vinylpyrrolidone copolymer include copolymers of vinylpyrrolidone-imidazole with a quaternary ammonium salt.

The present composition may contain one cationic polymer, or two or more cationic polymers. Preferably, the present composition comprises only one cationic polymer.

The concentration of the cationic polymer in the present composition is not particularly limited, and is, for example, preferably 0.00001 to 10% (w/v), more preferably 0.0001 to 5% (w/v), still more preferably 0.001 to 5% (w/v), furthermore preferably 0.01 to 5% (w/v), furthermore preferably 0.01 to 3% (w/v).

In the present invention, the polyvinylpyrrolidone is a polymer compound obtained by polymerizing N-vinyl-2-pyrrolidone, and is a type of cationic polymer. The K value of polyvinylpyrrolidone for use in the present invention is preferably 17 or more, more preferably 17 to 90, still more preferably 25 to 90, furthermore preferably 30 to 90. Examples of the polyvinylpyrrolidone include polyvinylpyrrolidone K17, polyvinylpyrrolidone K25, polyvinylpyrrolidone K30, polyvinylpyrrolidone K40, polyvinylpyrrolidone K50, polyvinylpyrrolidone K60, polyvinylpyrrolidone K70, polyvinylpyrrolidone K80, polyvinylpyrrolidone K85, polyvinylpyrrolidone K90 and polyvinylpyrrolidone K120. The K value of polyvinylpyrrolidone is a viscosity-characteristic value correlated to the molecular weight, and is determined by measuring a relative viscosity (25°C) with a capillary viscometer, and substituting the relative viscosity into the following Fikentscher equation (1).

[Expression 1]

$$K=\frac{1.5\log\eta_{rel}-1}{0.15+0.003c}+\frac{[300c\log\eta_{rel}+2(c+1.5\log\eta_{rel})]^{1/2}}{0.15c+0.003c^2} \qquad (1)$$

In equation (1), $\eta_{rel}$ is a viscosity of a polyvinylpyrrolidone aqueous solution relative to water, and c is a concentration (%) of polyvinylpyrrolidone in the polyvinylpyrrolidone aqueous solution.

In the present invention, one polyvinylpyrrolidone may be used singly, or any combination of two or more polyvinylpyrrolidones having different K values may be used.

The concentration of polyvinylpyrrolidone in the present composition is not particularly limited, and is, for example, preferably 0.0001 to 10% (w/v), more preferably 0.001 to 5% (w/v), still more preferably 0.01 to 5% (w/v), furthermore preferably 0.01 to 3% (w/v), especially preferably 0.1 to 3% (w/v).

If necessary, pharmaceutically acceptable additives may be further added to the composition using a technique that is widely used. Examples of the additives that may be selected and added if necessary include buffering agents such as sodium phosphate, sodium hydrogenphosphate, sodium dihydrogenphosphate, sodium acetate and epsilon-aminocaproic acid; tonicity agents such as calcium chloride, sodium chloride, potassium chloride and concentrated glycerin; stabilizing agents such as sodium edetate; surfactants such as polysorbate; antioxidants such as ascorbic acid; preservatives such as benzalkonium chloride and chlorhexidine gluconate; and pH adjustors such as hydrochloric acid and sodium hydroxide. These additives may be used singly, or any combination of two or more thereof may be used.

The pH of the present composition is not limited to a specific value as long as the pH is within a range of values that are pharmaceutically acceptable. The pH of the present composition is preferably 8 or less, more preferably in the range of 4 to 8, still more preferably in the range of 5 to 8, furthermore preferably in the range of 6 to 8, especially preferably about 7.

In the present invention, the "ophthalmic composition" refers to a composition for use in prevention and/or treatment of eye diseases. Examples of the dosage form of the present composition include eye drops, eye ointments,

injections and ointments (which can be administered to, for example, the eyelid skin), with eye drops being preferable. Here, the eye drop is synonymous with an ophthalmic solution or an ophthalmic drug, and eye drops for contact lenses are also within the definition of eye drops.

The present composition is preferably an aqueous ophthalmic composition having water as a solvent (base), more preferably an aqueous eye drop.

The present composition may be a solution-type eye drop or a suspension-type eye drop depending on the nature, content, and the like of each of active ingredients and additives.

The viscosity of the present composition is adjusted to be preferably in the range of 1 to 500 mPa·s, more preferably in the range of 1 to 100 mPa·s, still more preferably in the range of 1 to 50 mPa·s, furthermore preferably in the range of 1 to 30 mPa·s, especially preferably in the range of 1 to 20 mPa·s, and measured with a rotary viscometer (25°C; a shear rate of 50 $s^{-1}$).

The osmotic pressure of the present composition is not limited to a specific value as long as the osmotic pressure is within a range of values that are pharmaceutically acceptable. The osmotic pressure of the present composition is preferably 2 or less, more preferably in the range of 0.5 to 2, still more preferably in the range of 0.7 to 1.6, furthermore preferably in the range of 0.8 to 1.4, especially preferably in the range of 0.9 to 1.2.

The present composition can be preserved in a state of being stored in a container made of any of various materials. For example, a container made of polyethylene, polypropylene or the like can be used. When the present composition is an eye drop, the present composition is stored in an eyedrop container, more specifically a "multidose-type eyedrop container" or a "unit dose-type eyedrop container".

In the present invention, the "multidose-type eyedrop container" refers to an eyedrop container including a container body, and a cap attachable to the container body, the eyedrop container allowing the cap to be freely detached and reattached. The multidose-type eyedrop container typically contains an ophthalmic solution in an amount equivalent to a plurality of doses so that the ophthalmic solution is used over a certain period of time. On the other hand, the "unit dose-type eyedrop container" refers to an eyedrop container in which a cap is welded to a bottle mouth portion with the intent of breaking the welded portion of the cap and a bottle-shaped body to detach the cap at the time of use. The unit dose-type eyedrop container contains an ophthalmic solution in an amount equivalent to one or several doses.

The dose regimen of the present composition can be appropriately changed according to the dosage form, the severity of a symptom of a patient to be dosed, the age and the body weight of the patient, the doctor’s judgment, and the like. For example, when an eye drop is selected as the dosage form, the present composition can be instilled into an eye 1 to 6 times a day, preferably 1 to 4 times a day, more preferably 1 to 2 times a day, at intervals of one day to one week, in a dose of 1 to 5 drops, preferably 1 to 3 drops, more preferably 1 to 2 drops, especially preferably 1 drop each time. Here, more specifically, the instillation frequency is, for example, preferably 6 times a day, 5 times a day, 4 times a day, 3 times a day, 2 times a day or 1 time a day, more preferably 6 times a day, 4 times a day, 3 times a day or 2 times a day, still more preferably 4 times a day or 3 times a day, especially preferably 3 times a day.

When the concentration of diquafosol or a salt thereof in the present composition is 3% (w/v), the present composition can be instilled into an eye 6 times a day, 5 times a day, 4 times a day, 3 times a day, 2 times a day or 1 time a day, preferably 6 times a day, 4 times a day, 3 times a day or 2 times a day, more preferably 4 times a day or 3 times a day, especially preferably 3 times a day, in a dose of 1 to 5 drops, preferably 1 to 3 drops, more preferably 1 to 2 drops, especially preferably 1 drop each time.

The amount of a drop is preferably about 0.1 to 30 μL, more preferably about 0.5 to 20 μL, still more preferably about 1 to 15 μL.

The present composition is effective for prevention or treatment of dry eye. The dry eye is defined as a "chronic lacrimal and keratoconjunctival epithelial disease which is attributed to various factors and which involves ocular discomfort or visual abnormality", and the dry eye includes keratoconjunctivitis sicca (KCS). In the present invention, the dry eye also includes development of dry eye symptoms caused by wear of soft contact lenses.

The dry eye symptoms include subjective symptoms such as a feeling of ocular dryness, ocular discomfort, a feeling of ocular fatigue, a dull feeling, a photophobic feeling, ocular pain and blurred vision (filmy vision), and objective findings such as bloodshot eyes and keratoconjunctival epithelial disorders.

While there are many unclear points regarding causal factors of dry eye, the causes of the dry eye have been reported to be Sjogren’s syndrome; congenital alacrima; sarcoidosis; graft versus host disease (GVHD) resulting from bone-marrow transplantation; ocular pemphigoid; Stevens-Johnson syndrome; lacrimal occlusion caused by trachoma etc.; diabetes; decreased reflex secretion caused by laser-assisted in situ keratomileusis etc.; meibomian gland dysfunction; oily layer reduction caused by blepharitis etc.; incomplete eyeblinking or incomplete eyelid closure caused by bulging of eyeballs, rabbit eye, etc.; decreased mucin secretion from embryonic cells; and operations with visual display terminals.

The present composition can be instilled into an eye to the eyes of a dry eye patient wearing soft contact lenses. Here, the instillation to the eyes of a dry eye patient wearing soft contact lenses means that the ophthalmic solution is instilled into an eye with soft contact lenses placed on the cornea of the dry eye patient.

EXAMPLES

The results of pharmacological tests and Formulation Examples will be shown below, and these examples are intended to provide a better understanding of the present invention, and should not be construed as limiting the scope of the present invention.

[Test 1]

Using normal male white rabbits, a time-dependent change in tear volume after instillation of an ophthalmic composition comprising diquafosol sodium and a cationic polymer was evaluated.

(Method for Preparing Drug)

Ophthalmic solution 1:

An ophthalmic solution 1 was prepared in accordance with the formulation table shown in Table 1 (in Table 1, the concentration of each ingredient is measured in g/100 mL). Specifically, 3 g of diquafosol sodium, 1 g of calcium chloride ($CaCl_2$) and 40 mL of a solution of chitosan (70/200) (Chitosan 70/200 (Product No. 24205) from Product Line: Chitoceuticals of HEPPE MEDICAL CHITOSAN GmBH) were dissolved in sterile purified water, a pH adjustor was added, and the amount of the solution was adjusted to 100 mL to prepare ophthalmic solution 1. It is to be noted that the solution of chitosan (70/200) was prepared in the following manner: 1.5 g of chitosan (70/200) was dissolved in sterile purified water made acidic with dilute hydrochloric acid while heating was performed, a pH adjustor was added, and the amount of the solution was adjusted to 100 mL. Here, the chitosan (70/200) refers to chitosan having a deacetylation of deacetylation of 70% and a viscosity estimate of about 200 mPa·s in terms of a 1% aqueous solution.

Ophthalmic solutions 2 and 3:

Similarly to ophthalmic solution 1, each of ophthalmic solutions 2 and 3 was prepared in accordance with the formulation table shown in Table 1.

[Table 1]

| Ophthalmic solution | 1 | 2 | 3 |
|---|---|---|---|
| Diquafosol sodium | 3 | 3 | - |
| Chitosan (70/200) | 0.6 | 0.6 | 0.6 |
| Calcium chloride | 1.0 | 0.6 | 1.0 |
| pH Adjustor | q.s. | q.s. | q.s. |
| pH | 6.4 | 6.6 | 6.2 |
| Osmosis pressure ratio | 1 | 1 | 1 |
| Viscosity (mPa·s) | 16.8 | 2.4 | 13.8 |

Ophthalmic solution 4:

As an ophthalmic solution 4, "Diquas (registered trademark) Ophthalmic Solution 3%" (manufactured by Santen Pharmaceutical Co., Ltd.) available as a therapeutic agent for dry eye was used. Ophthalmic solution 4 contains 30 mg of diquafosol sodium as an active ingredient and potassium chloride, sodium chloride, a chlorhexidine gluconate solution, sodium hydrogenphosphate hydrate, sodium edetate hydrate and a pH adjustor as additives in 1 mL of water.

Ophthalmic solution 5:

As an ophthalmic solution 5, the base of ophthalmic solution 4 was used.

It is to be noted that the viscosities of prepared ophthalmic solutions 1 to 3 were measured at a temperature of 25°C and a shear rate of 50 $s^{-1}$ using a rotary viscometer Kinexus pro+.

(Test Method and Method for Administering Drug)

Benoxil (registered trademark) Ophthalmic Solution 0.4% (manufactured by Santen Pharmaceutical Co., Ltd.) was instilled into an eye to normal male white rabbits (total 44 rabbits with 88 eyes), and topical anesthesia was applied. After three minutes, a Schirmer test strip (manufactured by AYUMI Pharmaceutical Corporation) was inserted into the lower eyelid. One minute after the insertion, the test strip was removed, and the length of a wet portion (tear volume) was read. This value was defined as a pre-instillation value. Subsequently, ophthalmic solutions 1 to 5 were each instilled into an eye once (4 rabbits with 8 eyes per group). Three minutes before a Schirmer test strip (manufactured by AYUMI Pharmaceutical Corporation) was inserted into the lower eyelid, Benoxil (registered trademark) Ophthalmic Solution 0.4% (manufactured by Santen Pharmaceutical Co., Ltd.) was instilled into an eye, and topical anesthesia was applied. 30 minutes after the instillation of the ophthalmic solutions, a Schirmer test strip (manufactured by AYUMI Pharmaceutical Corporation) was inserted into the lower eyelid. One minute after the insertion, the test strip was removed, and the length of a wet portion (tear volume) was read.

(Evaluation Method)

A change between tear volume before and tear volume after instillation of the ophthalmic solution was calculated as Δ tear volume (mm/minute).

(Test Results)

Table 2 shows Δ tear volume (mm/minute) at each ophthalmic solution. Each value is an average value for 8 eyes.

[Table 2]

| | Δ Tear volume 30 minutes after instillation (mm/minute) |
|---|---|
| Ophthalmic solution 1 | 12.38 |
| Ophthalmic solution 2 | 8.94 |
| Ophthalmic solution 3 | 3.06 |
| Ophthalmic solution 4 | 4.19 |
| Ophthalmic solution 5 | 0.63 |

(Discussions)

When chitosan which is a cationic polymer was used as an additive (ophthalmic solutions 1 and 2), high Δ tear volume was exhibited even 30 minutes after the instillation, and therefore an ophthalmic composition comprising diquafosol sodium and a cationic polymer was shown to have an excellent tear volume increasing action.

[Test 2]

As in Test 1, the tear volume after instillation of an ophthalmic composition comprising diquafosol sodium and a cationic polymer was evaluated using normal male white rabbits.

(Method for Preparing Drug)

Ophthalmic solution 6:

An ophthalmic solution 6 was prepared in accordance with the formulation table shown in Table 3 (in Table 3, the concentration of each ingredient is measured in g/100 mL). Specifically, 3 g of diquafosol sodium, 1 g of chitosan (oligomer) (Chitosan Oligomer (Product No. 44009) from Product Line: Chitoceuticals of HEPPE MEDICAL CHITOSAN GmBH) and 0.51 g of sodium chloride were dissolved in sterile purified water, the amount of the solution was adjusted to 100 mL, and a pH adjustor was added to prepare ophthalmic solution 6. The chitosan (oligomer) used for ophthalmic solution 6 has a degree of deacetylation of 75% or more and a viscosity estimate of about 5 mPa·s in terms of a 1% aqueous solution.

Ophthalmic solutions 7 and 8:

Similarly to ophthalmic solution 6, each of ophthalmic solutions 7 and 8 was prepared in accordance with the formulation table shown in Table 3. Chitosan N-acetylcysteine (Kitopure N-Acetyl-Cysteine Conjugated Chitosan (Catalog No. KITO-7) from Poly Sci Tech (registered trademark) Company) used for ophthalmic solutions 7 and 8 has a degree of deacetylation of 75 to 85% or more and a viscosity estimate of about 5 mPa·s in terms of a 1% aqueous solution.

[Table 3]

| Ophthalmic solution | 6 | 7 | 8 |
|---|---|---|---|
| Diquafosol sodium | 3 | 3 | - |
| Chitosan (oligomer) | 1 | - | - |
| Chitosan N-acetylcysteine | - | 1 | 1 |
| NaCl | 0.51 | 0.11 | 0.45 |
| pH Adjustor | q.s. | q.s. | q.s. |
| pH | 6 | 5.7 | 5.7 |
| Osmosis pressure ratio | 1 | 1 | 1 |
| Viscosity (mPa·s) | Not measured | Not measured | Not measured |

Ophthalmic solutions 4 and 5:

Ophthalmic solutions 4 and 5 shown in Test 1 were also used.

(Test Method and Method for Administering Drug)

Benoxil (registered trademark) Ophthalmic Solution 0.4% (manufactured by Santen Pharmaceutical Co., Ltd.) was instilled into an eye to normal male white rabbits (total 18 rabbits with 36 eyes), and topical anesthesia was applied. After three minutes, a Schirmer test strip (manufactured by AYUMI Pharmaceutical Corporation) was inserted into the lower eyelid. One minute after the insertion, the test strip was removed, and the length of a wet portion (tear volume) was read. This value was defined as a pre-instillation value.

Subsequently, ophthalmic solutions 4 to 8 were each instilled into an eye once (3 rabbits with 6 eyes or 4 rabbits with 8 eyes per group). Three minutes before a Schirmer test strip (manufactured by AYUMI Pharmaceutical Corporation) was inserted into the lower eyelid, Benoxil (registered trademark) Ophthalmic Solution 0.4% (manufactured by Santen Pharmaceutical Co., Ltd.) was instilled into an eye, and topical anesthesia was applied. 30 minutes after the instillation of the ophthalmic solutions, a Schirmer test strip (manufactured by AYUMI Pharmaceutical Corporation) was inserted into the lower eyelid. One minute after the insertion, the test strip was removed, and the length of a wet portion (tear volume) was read.

(Evaluation Method)

A change between tear volume before and tear volume after instillation of the ophthalmic solution was calculated as Δ tear volume (mm/minute).

(Test Results)

Table 4 shows Δ tear volume (mm/minute) at each ophthalmic solution. Each value is an average value for 6 or 8 eyes.

[Table 4]

| | Δ Tear volume 30 minutes after instillation (mm/minute) |
|---|---|
| Ophthalmic solution 6 | 7.44 |
| Ophthalmic solution 7 | 7.38 |

(continued)

| | Δ Tear volume 30 minutes after instillation (mm/minute) |
|---|---|
| Ophthalmic solution 8 | -0.92 |
| Ophthalmic solution 4 | 3.25 |
| Ophthalmic solution 5 | 0.92 |

(Discussions)

When chitosan or chitosan N-acetylcysteine which is a cationic polymer was used as an additive (ophthalmic solutions 6 and 7), high Δ tear volume was exhibited even 30 minutes after the instillation, and therefore an ophthalmic composition comprising diquafosol sodium and a cationic polymer was shown to have an excellent tear volume increasing action.

[Test 3]

Effects of the additives on the metabolic stability of diquafosol sodium were examined.

(Method for Preparing Sample)

Ophthalmic solution 9:

An ophthalmic solution 9 was prepared in accordance with the formulation table shown in Table 5 (in Table 5, the concentration of each ingredient is measured in g/100 mL). Specifically, 1 g of diquafosol sodium, 3.22 g of sodium chloride and 80 mL of a solution of chitosan (Chitosan low molecular weight (Catalog No. 448869) from SIGMA ALDRICH) were dissolved in sterile purified water, a pH adjustor was added, and the amount of the solution was adjusted to 100 mL to prepare ophthalmic solution 9. It is to be noted that the solution of chitosan was prepared in the following manner: 1 g of chitosan was dissolved in sterile purified water made acidic with dilute hydrochloric acid while heating was performed, a pH adjustor was added, and the amount of the solution was adjusted to 100 mL. Here, the chitosan used for ophthalmic solution 9 has a degree of deacetylation of 75 to 85% and a viscosity estimate of about 20 mPa·s to about 300 mPa·s in terms of a 1% aqueous solution.

Ophthalmic solutions 10 to 20:

Similarly to ophthalmic solution 9, each of ophthalmic solutions 10 to 20 was prepared in accordance with the formulation table shown in Table 5.

[Table 5]

| Ophthalmic solution | 9 | 10 | 11 | 12 | 13 | 14 | 15 | 16 | 17 | 18 | 19 | 20 |
|---|---|---|---|---|---|---|---|---|---|---|---|---|
| Diquafosol sodium | 1 | 1 | 1 | 1 | 1 | 1 | 1 | 1 | 1 | 1 | 1 | 1 |
| Chitosan | 0.8 | - | - | - | - | - | - | - | - | - | - | - |
| Agmatine sulfate | - | 2.28 | - | - | - | - | - | - | - | - | - | - |
| Spermine | - | - | 2.02 | - | - | - | - | - | - | - | - | - |
| Trometamol | - | - | - | 1.21 | - | - | - | - | - | - | - | - |
| ε-Aminocaproic acid | - | - | - | - | 1.31 | - | - | - | - | - | - | - |
| Meglumine | - | - | - | - | - | 1.95 | - | - | - | - | - | - |
| L-Arginine hydrochloride | - | - | - | - | - | - | 2.11 | - | - | - | - | - |
| Boric acid | - | - | - | - | - | - | - | 0.62 | - | - | - | - |
| Poly-L-lysine | - | - | - | - | - | - | - | - | 1.25 | - | - | - |
| Lysine | - | - | - | - | - | - | - | - | - | 1.46 | - | - |
| Ethyl enediaminetetraacetic acid | - | - | - | - | - | - | - | - | - | - | 5 | - |
| Sodium chloride | 3.22 | - | - | - | - | - | - | - | - | - | - | - |
| pH Adjustor | q.s. | q.s. | q.s. | q.s. | q.s. | q.s. | q.s. | q.s. | q.s. | q.s. | q.s. | - |
| pH | 5.7 | 7.9 | 7.9 | 7.9 | 7.9 | 7.9 | 7.9 | 7.9 | 7.9 | 7.9 | 7.9 | Not measured |

(Test Method)

90 μL of rabbit blood plasma and 90 μL of purified water were kept at 37°C with an incubator, and in this state, 10 μL of each of ophthalmic solutions 9 to 20 was mixed with 90 μL of the rabbit blood plasma and 90 μL of the purified water. The mixture was reacted for 6 hours, 0.3 mL of a 10% formic acid solution was then added, and the mixture was adequately vortexed. Thereafter, 100 μL of the reaction liquid was taken, and mixed with 900 μL of a 800 mL potassium phosphate/methanol solution, and the mixture was filtered using a 0.45 μm filter. The filtrate was analyzed by HPLC.

(Evaluation Method)

The residual ratio and the degradation inhibition ratio of the diquafosol sodium were calculated from the HPLC analysis result. The residual ratio and the degradation inhibition ratio were calculated from the following expressions.

- Residual ratio (%) = (concentration of diquafosol sodium in blood plasma/concentration of diquafosol in water) × 100

- Degradation inhibition ratio (%) = (residual ratio of each of ophthalmic solutions 9 to 19/residual ratio of ophthalmic solution 20) × 100

(Test Results)

Table 6 shows the residual ratios and the degradation inhibition ratios of the ophthalmic solutions.

[Table 6]

| Ophthalmic solution | Additive | Residual ratio (%) | Degradation inhibition ratio (times) |
|---|---|---|---|
| Ophthalmic solution 9 | Chitosan | 44 | 1.6 |
| Ophthalmic solution 10 | Agmatine sulfate | 24 | 0.9 |
| Ophthalmic solution 11 | Spermine | 27 | 1.0 |
| Ophthalmic solution 12 | Trometamol | 26 | 1.0 |
| Ophthalmic solution 13 | ε-Aminocaproic acid | 26 | 1.0 |
| Ophthalmic solution 14 | Meglumine | 27 | 1.0 |
| Ophthalmic solution 15 | L-Arginine hydrochloride | 26 | 1.0 |
| Ophthalmic solution 16 | Boric acid | 36 | 1.3 |
| Ophthalmic solution 17 | Poly-L-lysine | 13 | 0.5 |
| Ophthalmic solution 18 | Lysine | 25 | 0.9 |
| Ophthalmic solution 19 | Ethylenediaminetetraacetic acid | 11 | 0.4 |
| Ophthalmic solution 20 | None | 27 | 1.0 |

(Discussions)

When chitosan which is a cationic polymer was used as an additive, metabolic degradation of diquafosol sodium was suppressed, and therefore the cationic polymer was shown to have a metabolic stability effect on diquafosol sodium.

[Test 4]

The stimulatory property of diquafosol sodium to peripheral nerves in the presence of PVP which is a type of cationic polymer was examined. "PVP" denotes polyvinylpyrrolidone. "CMC-Na" denotes carboxymethylcellulose sodium.
"HPMC" denotes hydroxypropylmethylcellulose. "CVP" denotes a carboxyvinyl polymer.

(Method for Preparing Sample)

Formulation solution 1:

A formulation solution 1 was prepared in accordance with the formulation table shown in Table 7 (in Table 7, the concentration of each ingredient is measured in g/100 mL). Specifically, sodium chloride (8.5 g) and sodium hydrogenphosphate hydrate (2 g) were dissolved in sterile purified water, a pH adjustor was added to adjust the pH to 7.5, and the total amount was then adjusted to 100 mL to give a 10-fold buffer solution. PVP K30 (16 g) was dissolved in sterile purified water, and the total amount was adjusted to 200 mL to give a 8% PVP K30 aqueous solution. 2 mL of the 10-fold buffer solution and 5 mL of the 8% PVP K30 aqueous solution were weighed and taken, the total amount was adjusted to 20 mL with sterile purified water, and the pH was adjusted to 7.5 with a pH adjustor to give formulation solution 1.

Formulation solution 2:

A formulation solution 2 was prepared in accordance with the formulation table shown in Table 7. Specifically, sodium chloride (8.5 g) and sodium hydrogenphosphate hydrate (2 g) were dissolved in sterile purified water, a pH adjustor was added to adjust the pH to 7.5, and the total amount was then adjusted to 100 mL to give a 10-fold buffer solution. 2 mL of the 10-fold buffer solution and PVP K90 (0.4 g) were dissolved in sterile purified water, the PH was adjusted to 7.5 with a pH adjustor, and the total amount was adjusted to 20 mL to give formulation solution 2.

Formulation solution 3:

A formulation solution 3 was prepared in accordance with the formulation table shown in Table 7. Specifically, sodium chloride (8.5 g) and sodium hydrogenphosphate hydrate (2 g) were dissolved in sterile purified water, a pH adjustor was added to adjust the pH to 7.5, and the total amount was then adjusted to 100 mL to give a 10-fold buffer solution. 2 mL of the 10-fold buffer solution and sodium chondroitin sulfate (0.06 g) were added to sterile purified water, the PH was adjusted to 7.5 with a pH adjustor. After making sure that the solid component was dissolved, the total amount was adjusted to 20 mL to give formulation solution 3.

Formulation solutions 4 to 6:

Similarly to formulation solution 3, each of formulation solutions 4 to 6 was prepared in accordance with the formulation table shown in Table 7.

[Table 7]

| Formulation solution | 1 | 2 | 3 | 4 | 5 | 6 |
|---|---|---|---|---|---|---|
| Sodium chloride | 0.85 | 0.85 | 0.85 | 0.85 | 0.80 | 0.85 |
| Sodium hydrogenphosphate hydrate | 0.20 | 0.20 | 0.20 | 0.20 | 0.20 | 0.20 |
| PVP K30 | 2.00 | - | - | - | - | - |
| PVP K90 | - | 2.00 | - | - | - | - |
| Sodium chondroitin sulfate | - | - | 0.30 | - | - | - |
| HPMC | - | - | - | 0.30 | - | - |
| CVP | - | - | - | - | 0.30 | - |
| CMC-Na | - | - | - | - | - | 0.30 |
| pH Adjustor | q.s. | q.s. | q.s. | q.s. | q.s. | q.s. |
| pH | 7.5 | 7.5 | 7.5 | 7.5 | 7.5 | 7.5 |

(Test Method)

Cultured peripheral nerve cells (dorsal neuro ganglion neuron; purchased from Lonza Japan Ltd.) were incubated in a buffer solution containing an intracellular calcium indicator fluorescent dye (FLIPR Calcium 6 Assay Kit; Molecular Devices, LLC). 40% of the total amount of the buffer solution was replaced by each of the above-described formulation solutions. A non-stimulation group and a stimulation control group were similarly treated with the buffer solution instead of the formulation solution. The cells were left standing at room temperature, followed by starting fluorescence measurement over time with the calcium indicator dye using a fluorescence plate reader. 60 seconds after the start of the measurement, diquafosol sodium (final concentration: 0.3%) was added, and the measurement of fluorescence intensity was continued.

(Evaluation Method)

The maximum fluorescence intensity (RFUmax) after addition of diquafosol sodium was calculated as a relative value against the fluorescence intensity (RFU) immediately before the addition, with the fluorescence intensity (RFU) defined as 100%.

(Test Results)

Fig. 1 shows the results. For the stimulation control group and each of the groups treated with formulation solutions 3 to 6, a RFUmax of 103.5% or more was achieved with RFU increasing after addition of diquafosol sodium. On the other hand, in each of the groups treated with formulation solutions 1 and 2 containing PVP, the RFUmax was less than 101%.

(Discussions)

Peripheral nerve cells receiving some stimulation generates an action potential to fall into an excited state, and thereafter a stimulation signal converted into the action potential is transmitted to the central nerve system. The action potential is a cell membrane potential change caused by penetration of cations including calcium ions into cells. Thus, an increase in concentration of calcium ions in nerve cells is widely used experimentally as an indicator of an excited state of nerve cells. When peripheral nerve cells were exposed to diquafosol sodium, the fluorescence intensity of intracellular calcium ions quickly increased, and the nerve cells received diquafosol sodium as a stimulation to fall into an excited state. In each of the groups treated with polymer formulation solutions 3 to 6 which were categorized as comparative examples and which did not contain PVP, there was a similar stimulation response, and thus the polymers, i.e. sodium chondroitin sulfate, HPMC, CVP and CMC-Na, had no effect on the neurostimulatory property of diquafosol sodium. On the other hand, under formulation solutions 1 and 2 containing PVP, there was no increase in calcium ion signals in nerve cells after addition of diquafosol sodium. That is, diquafosol sodium has no neurostimulatory property in the presence of PVP, and thus addition of PVP improves the pouring touch of diquafosol sodium.

[Test 5]

Using normal male white rabbits, a time-dependent change in tear volume after instillation of the present composition was evaluated.

(Method for Preparing Drug)

Ophthalmic solution A:

An ophthalmic solution A was prepared in accordance with the formulation table shown in Table 8 (in Table 8, the concentration of each ingredient is measured in g/10 mL). Specifically, diquafosol sodium (9 g), sodium hydrogenphosphate hydrate (0.6 g), sodium edetate hydrate (0.03 g) and sodium chloride (1.35 g) were dissolved in sterile purified water, and the amount of the solution was adjusted to 50 mL to give a 6-fold concentrated solution. 10 mL of the 6-fold concentrated solution and 5 mL of sterile purified water were mixed, a pH adjustor was then appropriately added to adjust the pH to 7, and sterile purified water was added to 20 mL to give a 3-fold concentrated solution. PVP K90 (4 g) was dissolved in sterile purified water, the total amount was adjusted to 100 g, and high-pressure steam sterilization was then performed (at 121°C for 20 minutes) to give a 4.00% (w/w) PVP K90 solution. 4 mL of the 3-fold concentrated solution was added to 6.0 g of the 4.00% (w/w) PVP K90 solution, sterile purified water was added to adjust the total amount to 12 mL, and a pH adjustor was then appropriately added to adjust the pH to 7, thereby preparing ophthalmic

solution A.

Ophthalmic solution B:

An ophthalmic solution B was prepared in accordance with the formulation table shown in Table 8. Specifically, diquafosol sodium (9 g), sodium hydrogenphosphate hydrate (0.6 g), sodium edetate hydrate (0.03 g) and sodium chloride (1.35 g) were dissolved in sterile purified water, and the amount of the solution was adjusted to 50 mL to give a 6-fold concentrated solution. 10 mL of the 6-fold concentrated solution and 5 mL of sterile purified water were mixed, PVP K30 (1.2 g) was then dissolved, a pH adjustor was then appropriately added to adjust the pH to 7, and sterile purified water was added to 20 mL to give a 3-fold concentrated solution. To 4 mL of the 3-fold concentrated solution, sterile purified water was added to adjust the total amount to 12 mL, and a pH adjustor was then appropriately added to adjust the pH to 7, thereby preparing ophthalmic solution B.

Ophthalmic solution C:

An ophthalmic solution C was prepared in accordance with the formulation table shown in Table 8. Specifically, diquafosol sodium (9 g), sodium hydrogenphosphate hydrate (0.6 g), sodium edetate hydrate (0.03 g) and sodium chloride (1.35 g) were dissolved in sterile purified water, and the amount of the solution was adjusted to 50 mL to give a 6-fold concentrated solution. 10 mL of the 6-fold concentrated solution and 5 mL of sterile purified water were mixed, a pH adjustor was then appropriately added to adjust the pH to 7, and sterile purified water was added to 20 mL to give a 3-fold concentrated solution. To 4 mL of the 3-fold concentrated solution, sterile purified water was added to adjust the total amount to 12 mL, and a pH adjustor was then appropriately added to adjust the pH to 7, thereby preparing ophthalmic solution C.

It is to be noted that the viscosities of prepared ophthalmic solutions A to C were measured at a temperature of 25°C and a shear rate of 50 $s^{-1}$ using a rotary viscometer Kinexus pro+.

(Test Method and Method for Administering Drug)

Benoxil (registered trademark) Ophthalmic Solution 0.4% (manufactured by Santen Pharmaceutical Co., Ltd.) was instilled into an eye to normal male white rabbits (total 12 rabbits with 24 eyes), and topical anesthesia was applied. After three minutes, a Schirmer test strip (manufactured by AYUMI Pharmaceutical Corporation) was inserted into the lower eyelid. One minute after the insertion, the test strip was removed, and the length of a wet portion (tear volume) was read. This value was defined as a pre-instillation value. Subsequently, ophthalmic solutions A to C were each instilled into an eye once (4 rabbits with 8 eyes per group with the exception of ophthalmic solution C administered to 12 rabbits with 24 eyes). Three minutes before a Schirmer test strip (manufactured by AYUMI Pharmaceutical Corporation) was inserted into the lower eyelid, Benoxil (registered trademark) Ophthalmic Solution 0.4% (manufactured by Santen Pharmaceutical Co., Ltd.) was instilled into an eye, and topical anesthesia was applied. 60 minutes after the instillation of the ophthalmic solutions, a Schirmer test strip (manufactured by AYUMI Pharmaceutical Corporation) was inserted into the lower eyelid. One minute after the insertion, the test strip was removed, and the length of a wet portion (tear volume) was read.

(Evaluation Method)

A change between tear volume before and tear volume after instillation of the ophthalmic solution was calculated as Δ tear volume (mm/minute).

(Test Results)

Table 8 shows Δ tear volume (mm/minute) 60 minutes after instillation (each value is an average value for 8 eyes with the exception of ophthalmic solution C for which average values for 24 eyes are shown). The tear volume increasing action of the present composition was evaluated in accordance with the following criteria.
+++: Δ Tear volume (mm/minute) 60 minutes after instillation is 4 mm/minute or more.
++: Δ Tear volume 60 minutes after instillation (mm/minute) is 1 mm/minute or more and less than 4 mm/minute.
+: Δ Tear volume 60 minutes after instillation (mm/minute) is more than 0 mm/minute and less than 1 mm/minute.
-: Δ Tear volume 60 minutes after instillation (mm/minute) is 0 mm/minute or less.

[Table 8]

| Ophthalmic solution | A | B | C |
|---|---|---|---|
| Diquafosol sodium | 3 | 3 | 3 |
| PVP K30 | - | 2 | - |
| PVP K90 | 2 | - | - |
| Sodium hydrogenphosphate hydrate | 0.2 | 0.2 | 0.2 |
| Sodium edetate hydrate | 0.01 | 0.01 | 0.01 |
| Sodium chloride | 0.45 | 0.45 | 0.45 |
| pH adjustor | q.s. | q.s. | q.s. |
| pH | 7.0 | 7.0 | 7.0 |
| Viscosity (mPa·S) | 7.4 | 1.4 | 1.0 |
| Δ Tear volume 60 minutes after instillation (mm/minute) | 4.4 | 0.4 | 0.3 |
| Grade | +++ | + | + |

The results in Table 8 show that an ophthalmic solution (ophthalmic solution A) containing PVP which is a type of cationic polymer, particularly PVP K90, had a high tear volume increasing action.

[Preparation Examples]

The pharmaceutical agents of the present invention will be described in further detail by way of Preparation Examples, which should not be construed as limiting the present invention.

(Formulation Example 1: eye drop (3% (w/v))

In 100 mL:

Diquafosol sodium: 3 g
Chitosan: 0.6 g
Calcium chloride: 1.0 g
Sterile purified water: q.s.

The eye drop can be prepared by adding the diquafosol sodium and the other components to the sterile purified water, and sufficiently stirring the mixture.

(Formulation Example 2: eye drop (3% (w/v))

In 100 mL:

Diquafosol sodium: 3 g
Sodium hydrogenphosphate hydrate: 0.01 to 0.5 g
Sodium edetate hydrate: 0.0001 to 0.1 g
Polyvinylpyrrolidone: 0.0001 to 10 g
Sodium chloride: 0.01 to 1 g
pH adjuster: q.s.

The eye drop can be prepared by adding the diquafosol sodium and the other components to the sterile purified water, and sufficiently stirring the mixture.

INDUSTRIAL APPLICABILITY

The present composition has a high tear volume increasing action. Further, the cationic polymer contained in the present composition has a metabolic stability effect on diquafosol or a salt thereof. Hence, the present composition

is expected to exhibit a more potent therapeutic effect on dry eye as compared to a case where an existing Diquas (registered trademark) Ophthalmic Solution is instilled into an eye. The existing Diquas (registered trademark) Ophthalmic Solution is required to be instilled into an eye 6 times a day, and some patients are unable to obtain an expected effect due to poor adherence to instillation. The present composition is expected to improve adherence to instillation with the instillation frequency reduced while exhibiting a sufficient therapeutic effect on dry eye. Further, the existing Diquas (registered trademark) Ophthalmic Solution contains a diquafosol tetrasodium salt at a concentration of 3% (w/v), whereas the present composition is expected to exhibit an equivalent or more potent therapeutic effect on dry eye with a lower concentration. An ophthalmic composition comprising diquafosol or a salt thereof and polyvinylpyrrolidone which is a type of cationic polymer does not exhibit neurostimulatory, and therefore enables improvement of the pouring touch of the ophthalmic solution.

## Claims

1. An ophthalmic composition comprising diquafosol or a salt thereof and a cationic polymer, the cationic polymer being at least one selected from the group consisting of chitosan, a chitosan derivative, a cationic (meth)acrylate copolymer, a cationic silicone polymer, a diallyl quaternary ammonium salt-acrylamide copolymer, cationic hydrolyzed keratin, cationic hydrolyzed silk, cationic hydrolyzed collagen, cationic hydrolyzed casein, cationic hydrolyzed soy protein, a cationic vinylpyrrolidone copolymer, polyvinylpyrrolidone, a dimethyldiacrylammonium chloride homopolymer, an adipic acid-dimethylaminohydroxypropyldiethylenetriamine copolymer, an adipic acid-epoxypropyldiethylenetriamine copolymer and an acrylamide-β-methacryloyloxyethyltrimethylammoniummethyl sulfate copolymer.

2. The ophthalmic composition according to claim 1, wherein the cationic polymer is at least one selected from the group consisting of chitosan, a chitosan derivative, a diallyl quaternary ammonium salt-acrylamide copolymer and polyvinylpyrrolidone.

3. The ophthalmic composition according to claim 1 or 2, wherein the cationic polymer is polyvinylpyrrolidone.

4. The ophthalmic composition according to any one of claims 1 to 3, comprising polyvinylpyrrolidone having a K value of 17 or more

5. The ophthalmic composition according to any one of claims 1 to 4, comprising polyvinylpyrrolidone having a K value of 17 to 90.

6. The ophthalmic composition according to any one of claims 1 to 5, comprising polyvinylpyrrolidone having a K value of 30.

7. The ophthalmic composition according to any one of claims 1 to 6, comprising polyvinylpyrrolidone having a K value of 90.

8. The ophthalmic composition according to claim 1 or 2, wherein the cationic polymer is at least one selected from the group consisting of chitosan and a chitosan derivative.

9. The ophthalmic composition according to any one of claims 1 to 8, wherein the concentration of the cationic polymer is 0.00001 to 10% (w/v).

10. The ophthalmic composition according to any one of claims 1 to 9, wherein the concentration of the diquafosol or a salt thereof is 0.0001 to 10% (w/v).

11. The ophthalmic composition according to any one of claims 1 to 10, wherein the concentration of the diquafosol or a salt thereof is 0.01 to 5% (w/v).

12. The ophthalmic composition according to any one of claims 1 to 11, wherein the concentration of the diquafosol or a salt thereof is 1 to 5% (w/v).

13. The ophthalmic composition according to any one of claims 1 to 12, wherein the concentration of the diquafosol or a salt thereof is 3% (w/v).

**14.** The ophthalmic composition according to any one of claims 1 to 13, wherein the ophthalmic composition is an eye drop.

**15.** The ophthalmic composition according to any one of claims 1 to 14, wherein the ophthalmic composition is aqueous.

**16.** The ophthalmic composition according to any one of claims 1 to 15, wherein the ophthalmic composition is a suspension-type or solution-type composition.

**17.** The ophthalmic composition according to any one of claims 1 to 16, wherein the viscosity is 1 to 500 mPa·s at 25°C.

**18.** The ophthalmic composition according to any one of claims 1 to 17, wherein the viscosity is 1 to 100 mPa·s at 25°C.

**19.** The ophthalmic composition according to any one of claims 1 to 18, wherein the salt of diquafosol is diquafosol sodium.

**20.** The ophthalmic composition according to any one of claims 1 to 19, wherein the ophthalmic composition is a composition for prevention or treatment of dry eye.

**21.** The ophthalmic composition according to any one of claims 1 to 20, wherein the ophthalmic composition is **characterized by** being instilled into an eye 1 to 6 times a day in a dose of 1 to 5 drops each time.

**22.** The ophthalmic composition according to any one of claims 1 to 21, wherein the ophthalmic composition is **characterized by** being instilled into an eye 2 to 4 times a day in a dose of 1 or 2 drops each time.

**23.** The ophthalmic composition according to any one of claims 1 to 22, wherein the ophthalmic composition is **characterized by** being instilled into an eye 3 or 4 times a day in a dose of 1 or 2 drops each time.

**24.** An ophthalmic composition comprising diquafosol or a salt thereof and polyvinylpyrrolidone.

**25.** The ophthalmic composition according to claim 24, comprising polyvinylpyrrolidone having a K value of 17 or more.

**26.** The ophthalmic composition according to claim 24 or 25, comprising polyvinylpyrrolidone having a K value of 17 to 90.

**27.** The ophthalmic composition according to any one of claims 24 to 26, comprising polyvinylpyrrolidone having a K value of 30.

**28.** The ophthalmic composition according to any one of claims 24 to 27, comprising polyvinylpyrrolidone having a K value of 90.

FIG.1

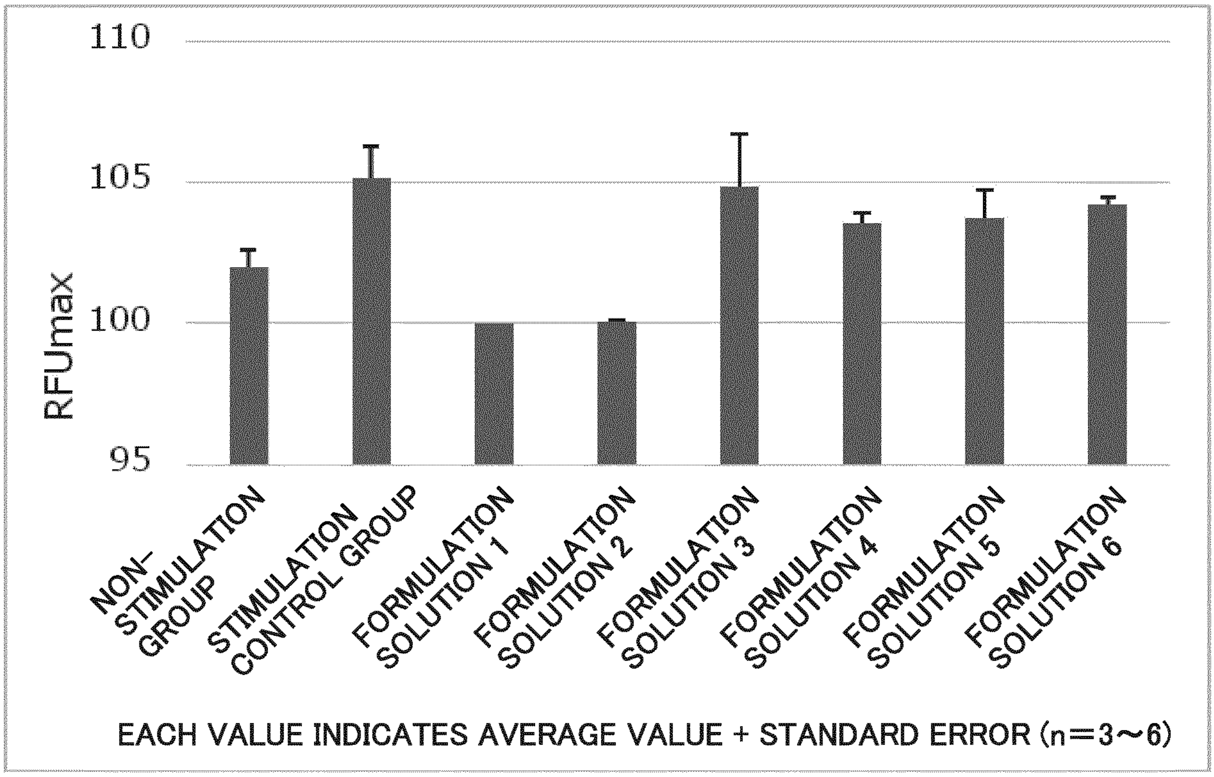
110
105
100
95
RFUmax
NON-STIMULATION GROUP
STIMULATION CONTROL GROUP
FORMULATION SOLUTION 1
FORMULATION SOLUTION 2
FORMULATION SOLUTION 3
FORMULATION SOLUTION 4
FORMULATION SOLUTION 5
FORMULATION SOLUTION 6
EACH VALUE INDICATES AVERAGE VALUE + STANDARD ERROR (n=3~6)

# INTERNATIONAL SEARCH REPORT

International application No.
PCT/JP2019/007542

A. CLASSIFICATION OF SUBJECT MATTER
Int.Cl. A61K31/7072(2006.01)i, A61K9/08(2006.01)i, A61K9/10(2006.01)i, A61K47/32(2006.01)i, A61K47/34(2017.01)i, A61K47/36(2006.01)i, A61K47/42(2017.01)i, A61P27/02(2006.01)i
According to International Patent Classification (IPC) or to both national classification and IPC

B. FIELDS SEARCHED
Minimum documentation searched (classification system followed by classification symbols)
Int.Cl. A61K31/7072, A61K9/08, A61K9/10, A61K47/32, A61K47/34, A61K47/36, A61K47/42, A61P27/02

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| Y<br>A | WO 2012/090994 A1 (SANTEN PHARMACEUTICAL CO., LTD.) 05 July 2012, claim 1, paragraph [0002], treatment example 1 & EP 2659894 A1 claim 1, paragraph [0002], treatment example 1 & US 2014/0221306 A1 & AU 2011350762 A & CA 2823148 A1 & CN 103282039 A & SG 191389 A & MX 2013007502 A & EA 201390985 A & KR 10-2014-0003493 A & BR 112013016008 A & TW 201306844 A & NZ 712814 A | 1-2, 8-23<br>3-7, 24-28 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 May 2019 (17.05.2019) | 04 June 2019 (04.06.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/007542

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | WO 2016/104704 A1 (SANTEN PHARMACEUTICAL CO., LTD.) 30 June 2016, claim 1, paragraph [0004], treatment example 1 & US 2017/0348344 A1 claim 1, paragraph [0004], treatment example 1 & JP 2016-210759 A & JP 2017-2036 A & WO 2016/195073 A1 & CA 2971689 A1 & KR 10-2017-0067900 A & KR 10-2017-0118255 A & EA 201791467 A & SG 11201705138Q A & MX 2017008277 A & KR 10-2018-0063381 A & BR 112017013841 A & TW 201628626 A | 1-2, 8-23<br>3-7, 24-28 |
| Y<br>A | WO 01/91795 A1 (SANTEN PHARMACEUTICAL CO., LTD.) 06 December 2001, claim 3, page 1, lines 14-26, page 2, lines 6-8, page 5, lines 17-27, example 1 & US 2003/0186927 A1 claim 3, paragraphs [0003]-[0004], [0017], example 1 & US 2006/0009415 A1 & EP 1428538 A1 & JP 2002-53492 A & AU 6064301 A & CA 2413928 A1 & BR 111297 A & KR 10-2003-0031489 A & CN 1431914 A | 1-2, 8-23<br>3-7, 24-28 |
| Y | JP 6-192130 A (UNION CARBIDE CHEMICALS&PLASTICS TECHNOLOGY CORPORATION) 12 July 1994, claims 1, 4, paragraphs [0011], [0035], examples 1, 3 & EP 590655 A1 claims 1, 5, page 2, lines 45-46, page 5, lines 16-21, examples 1, 3 & US 5358706 A & US 5645827 A & CA 2107301 A1 | 1-2, 8-23 |
| Y | JP 9-509166 A (CIBA GEIGY AG.) 16 September 1997, claim 1, page 14, line 15 to page 17, line 3, examples 14-15 & WO 1995/022315 A1 claim 1, page 9, line 7 to page 11, line 7, examples 14-15 & US 5422116 A & EP 744938 A1 & NO 963431 A & AU 1464995 A & NZ 278668 A & FI 963217 A & CA 2181715 A & MX 9603435 A & ZA 9501323 A & IL 112674 A & CY 2314 A & CN 1140988 A | 1-2, 8-23 |
| Y | JP 2017-514916 A (CROMA-PHARMA GESELLSCHAFT M.B.H.) 08 June 2017, claims 1, 18, example 12 & WO 2015/169728 A1 claims 1, 18, example 12 & US 2017/0224614 A1 & EP 3139903 A1 & AU 2015257874 A & CA 2947065 A1 & CN 106456536 A & KR 10-2017-0030471 A & AR 100334 A & MX 2016013710 A & BR 112016024454 A & RU 2016143341 A & SG 11201608402W A & CL 2016002815 A | 1-2, 8-23 |
| A | GÖBBELS, M. et al., "Corneal epithelial permeability of dry eyes before and after treatment with artificial tears", Ophthalmology, 1992, vol. 99, no. 6, pp. 873-878, abstract | 3-7, 24-28 |
| P, X | CN 108853016 A (GUANGDONG DAGUANG PHARMACEUTICAL CO., LTD.) 23 November 2018, claims 1, 6 (Family: none) | 1-2, 8-9, 14-16, 19-20 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2006321757 A
- JP 3652707 B
- JP H01294620 B
- JP 2001510484 A